# EUROPÄISCHE PATENTSCHRIFT

(11) **EP 0 313 799 B1**
(45) Veröffentlichungstag und Bekanntmachung des Hinweises auf die Patenterteilung: **10.02.1993**
(21) Anmeldenummer: 88115306.8
(22) Anmeldetag: 19.09.1988
(51) Int. Cl.: C07C 217/18

(54) **Verfahren zur Herstellung von trans-1,1,2-Triphenyl -but-1-en Derivaten**
Process for the preparation of trans-1,1,2-triphenyl-but-1-ene derivatives
Procédé pour la préparation de dérivés du trans-1,1,2-triphényl-but-1-ène

(30) Priorität: 29.10.1987 DE 3736682
(43) Veröffentlichungstag der Anmeldung: 03.05.1989
(73) Patentinhaber: Klinge Pharma GmbH, D-81673 München (DE)
(72) Erfinder: Woschina, Axel, D-8011 Poing (DE); Grill, Helmut, Dr., D-8011 Vaterstetten (DE)
(74) Vertreter: Kolb, Helga, Dr. Dipl.-Chem.

(56) Entgegenhaltungen:
- EP-A- 0 054 168
- EP-A- 0 127 128
- EP-A- 0 168 175
- DE-A- 1 568 834
- CHEMICAL ABSATRACTS, Band 110, 24. April 1989, Zusammenfassung Nr. 153903m, Columbus, Ohio, US; & CN-A-86 107 840

## Beschreibung

In den letzten Jahren ist eine Reihe von Triphenylbuten-Derivaten beschrieben worden, die sich auf Grund ihrer antiestrogenen Eigenschaften zur Behandlung des hormonabhängigen Mammatumors eignen .[R.Sutherland and V.C. Jordan, "Nonsteroidal Antiestrogens" Academic Press,1981]
Eingang in die Therapie hat der Wirkstoff 1-[4'-(2-Dimethylaminoethoxy)phenyl]-trans-1,2-diphenyl-but-1-en gefunden, der inzwischen weltweit unter der Bezeichnung TAMOXIFEN (INN) bekannt geworden ist.

Bei der Synthese von 1,1,2-Triphenyl-but-1-en-Derivaten fallen in der Dehydratisierungsstufe der diastereomeren Carbinole der Formel 2 die geometrisch isomeren Olefine der Formeln 3 und 1 [cis/trans- bzw. E/Z-Form] im Gemisch an. 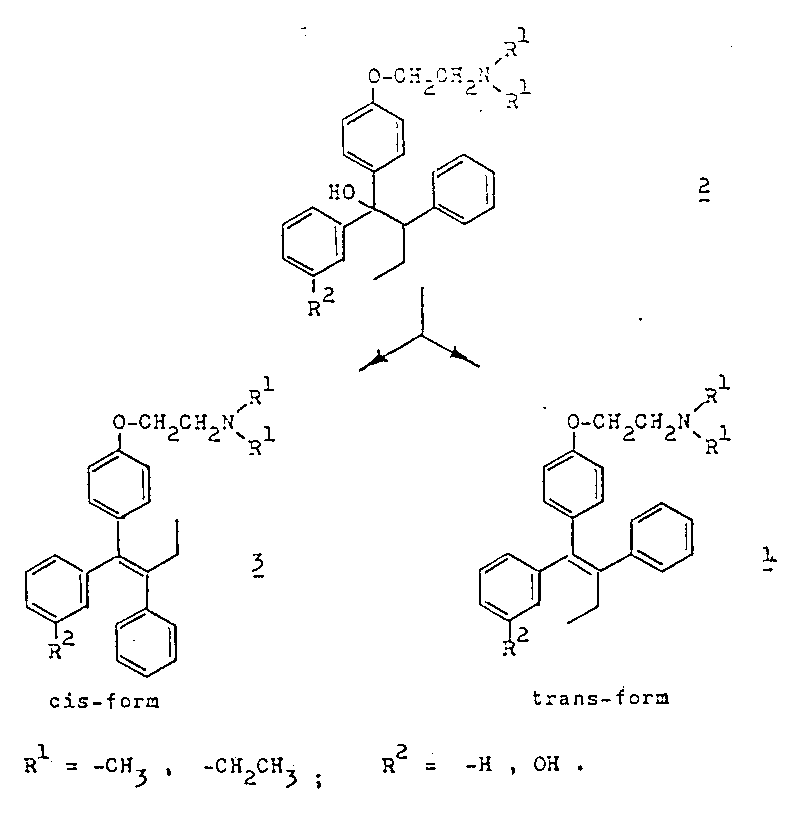
Da nur das wirksame Isomere für eine klinische Anwendung in Frage kommt, war es notwendig aus dem anfallenden Isomerengemisch die reine trans-Form zu isolieren. Dies geschah bisher nach aufwendigen , verlustreichen Verfahren, wie fraktionierte Kristallisation oder Chromatographie. [ UK-Patent No. 1 013 907; US-Patent No. 4 536 516 ; Europ.Patent No. 0 054 168; G.R. Bedford and D.N. Richardson , Nature 212, 733 - 734 (1966); P. Sohar. et al, Acta Chim.Acad.Sci.Hung. 100 69 - 74 (1979);D.W.Robertson and J.A. Katzenellenbogen J.Org.Chem. 47, 2387 - 2393 (1982) ; P.C. Ruenitz et al , J.Med. Chem. 25, 1056 - 1060 (1982) ; R.D.Armstrong, J.Chromatogr. 414 , 192 - 196 (1987)].

Die unbefriedigende Ausbeute an Tamoxifen konnte erst gesteigert werden, als es gelang, die bisher wertlosen, mit der cis-Form angereicherten Mutterlaugen-Fraktionen einer Umwandlung in die trans-Form zu unterziehen. Wie aus dem Europ.Patent No. 0 127 128 hervorgeht, läßt sich die cis-Form in stark salzsaurem Milieu bei erhöhter Temperatur in die trans-form umwandeln.

Überraschenderweise konnte jetzt eine wesentliche Vereinfachung im Verfahren zur Herstellung von trans-1,1,2-Triphenyl-but-1-en-Derivaten der allgemeinen Formel 1 erzielt werden. Wie gezeigt wird, lassen sich Carbinolverbindungen der allgemeinen Formel 2 unter bestimmten Bedingungen in einem Schritt weitgehend in die trans-Olefine der allgemeinen Formel 1 überführen. Damit entfallen die in den bisherigen Herstellungsverfahren notwendigen Stufen : a) Isolierung des cis/trans-Isomerengemisches nach Dehydratisierung der diastereomeren Carbinole, b) Abtrennung der trans-Form durch Kristallisation oder Chromatographie und c) Rückisomerisierung der mit der cis-Form angereicherten Mutterlaugen-Fraktionen.

Die Erfindung betrifft ein Verfahren zur Herstellung von trans-1,1,2-Triphenyl-but-1-en-Derivaten der allgemeinen Formel 1 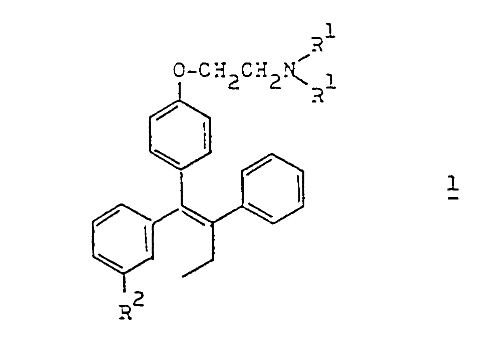
in der R¹ = CH₃, CH₂CH₃ und R² = H, OH,
welches dadurch gekennzeichnet ist, daß man Carbinole der allgemeinen Formel 2 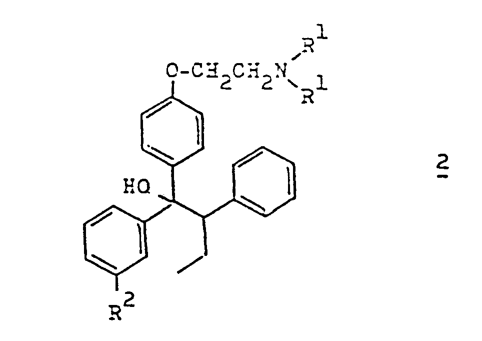
in der R¹ und R² die in der Formel 1 angegebene Bedeutung besitzen, mindestens 10 h bei einer Temperatur von 50° bis 60°C in mindestens 25 Gew.-% salzsaurem bzw. mindestens 40 Vol.-% schwefelsaurem Milieu unter Ausschluß von organischen Lösungsmitteln erhitzt und das Reaktionsprodukt nach üblichen Methoden aufarbeitet.

Im erfindungsgemäßen Verfahren werden Carbinole der allgemeinen Formel 2 in Abwesenheit von organischen Lösungsmitteln durch Einwirken von Salz- oder Schwefelsäure bei erhöhter Temperatur direkt in die trans-Olefine der Formel 1 überführt. Unter diesen Bedingungen wird eine Bildung von cis-Olefinen der Formel 3 praktisch unterdrückt.

Die Umwandlung der Carbinole gemäß Formel 2 in die trans-Olefine der Formel 1 erfolgt vorzugsweise im Temperaturbereich von 50 bis 60°C. Man läßt Salz- bzw. Schwefelsäure bei erhöhter Temperatur vorzugsweise 12 bis 16 h, mindestens jedoch 10 h lang einwirken.

Für die Umwandlung der Carbinole soll die Salzsäurekonzentration mindestens 25 Gew.-%, bevorzugt 32 bis 37 Gew.-% betragen. Im Falle der Verwendung von Schwefelsäure soll deren Konzentration mindestens 40 Vol.-%, vorzugesweise 45 bis 50 Vol.-% betragen.

Im folgenden wird die Erfindung anhand von Ausführungsbeispielen näher erläutert.

### Beispiel 1

1 Teil 1-[4'-(2-Dimethylaminoethoxy)phenyl]-1,2-diphenyl-butan-1-ol wird in 10 Teile 50-vol.proz. Schwefelsäure eingerührt und die Suspension 14 Stunden unter intensivem Rühren auf 55°C erwärmt. Anschließend wird abgekühlt und unter Hinzufügen von 2.5 Teilen Eis mit 12.5 Teilen konz. Ammoniak alkalisiert. Das Reaktionsprodukt wird in Ethylacetat aufgenommen und die organische Phase mehrmals mit Wasser gewaschen. Nach dem Entfernen des organischen Lösungsmittels im Vakuum verbleiben 0.9 Teile Rückstand mit einem Gehalt an 1-[4'-(2-Dimethylaminoethoxy)phenyl]-trans-1,2-diphenyl-but-1-en von 94% [HPLC]. Kristalle aus Aceton mit einem Schmp. von 98°C. Gehalt : 99.4 % [HPLC].

### Beispiel 2

1 Teil 1-[4′-(2-Dimethylaminoethoxy)phenyl]-1,2-diphenyl-butan-1-ol wird in 6 Teile 32-Gew.% Salzsäure eingerührt und die Suspension 16 Stunden unter intensivem Rühren auf 52° C erwärmt. Anschließend wird abgekühlt und unter Hinzufügen von 2 Teilen Eis mit 6 Teilen konz. Ammoniak alkalisiert. Das Reaktionsprodukt wird in Ethylacetat aufgenommen und die organische Phase mehrmals mit Wasser gewaschen. Nach dem Entfernen des organischen Lösungsmittels im Vakuum, verbleiben o.97 Teile Rückstand mit einem Gehalt an 1-[4′-(2-Dimethylaminoethoxy)phenyl]-trans-1,2-diphenyl-but-1-en von 96 % [HPLC]. Kristalle aus Methanol/Wasser; Schmp.96-98°C;Gehalt : 99.7 % [HPLC]

### Beispiel 3

1 Teil 1-[4′-(2-Dimethylaminoethoxy)phenyl]-1-(3′-hydroxyphenyl)--2-phenyl-butan-1-ol wird in 9 Teile 50- Vol. %. Schwefelsäure eingerührt und die Suspension 15 Stunden unter intensivem Rühren auf 52° C erwärmt. Anschließend wird abgekühlt und unter Hinzufügen von 3 Teilen Eis mit 12 Teilen konz. Ammoniak alkalisiert. Das Reaktionsprodukt wird in Dichlormethan aufgenommen und die organische Phase mehrmals mit Wasser gewaschen. Nach dem Entfernen des organischen Lösungsmittels in Vakuum verbleiben 0.9 Teile Rückstand mit einem Gehalt an 1-[4′-(2-Dimethylaminoethoxy)phenyl]-trans-1-(3′-hydroxyphenyl)-2-phenyl-but-1-en von 90 % [HPLC]. Kristalle aus Ethanol; Schmp. 164° C; Gehalt : 99.5 % [HPLC].

### Beispiel 4

1 Teil 1-[4′-(2-Dimethylaminoethoxy)phenyl]-1-(3′-hydroxyphenyl)-2-phenyl-butan-1-ol wird in 6 Teile 37-Gew. % Salzsäure eingerührt und die Suspension 16 Stunden unter intensivem Rühren auf 50° C erwärmt. Anschließend wird abgekühlt und unter Hinzufügen von 3 Teilen Eis mit 4 Teilen konz. Ammoniak alkalisiert. Das Reaktionsprodukt wird in Dichlormethan aufgenommen und die organische Phase mehrmals mit Wasser gewaschen. Nach dem Entfernen des organischen Lösungsmittels im Vakuum verbleiben 0.85 Teile Rückstand mit einem Gehalt an 1-[4′-(2-Dimethylamonoethoxy)phenyl]-trans-1-(3′-hydroxyphenyl)-2-phenyl-but-1-en von 93 % [HPLC]. Kristalle aus Ethanol; Schmp. 164°C; Gehalt : 99.6 % [HPLC].

### Beispiel 5

1 Teil 1-[4′-(2-Dimethylaminoethoxy)phenyl]-1-(3′-hydroxyphenyl)-2-phenyl-butan-1-ol wird in 8 Teile 37-Gew. % Salzsäure eingerührt und die Suspension 15 Stunden unter intensivem Rühren auf 52° C erwärmt. Anschließend wird abgekühlt und unter Hinzufügen von 4 Teilen Eis mit 5 Teilen konz. Ammoniak alkalisiert. Das Reaktionsprodukt wird in Dichlormethan aufgenommen und die organische Phase mehrmals mit Wasser gewaschen. Nach dem Entfernen des organischen Lösungsmittels im Vakuum verbleiben 0.95 Teile Rückstand mit einem Gehalt an 1-[4′-(2-Dimethylamonoethoxy)phenyl]-trans-1-(3′-hydroxyphenyl)-2-phenyl-but-1-en von 95 % [HPLC]. Kristalle aus Isopropanol ; Schmp. 130° C ; Gehalt : 99.5 % [HPLC] .

## Patentansprüche

1. Verfahren zur Herstellung von trans-1,1,2-Triphenyl-but-1-en-Derivaten der allgemeinen Formel 1 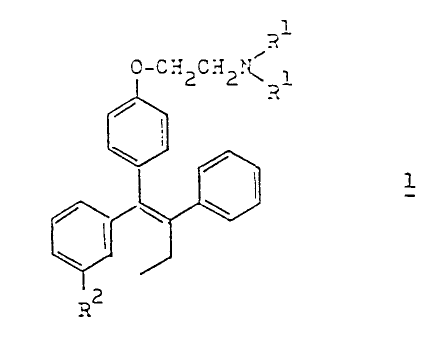 in der R¹ = CH₃, CH₂CH₃ und R² = H, OH
dadurch **gekennzeichnet,** dass man Carbinole der allgemeinen Formel 2 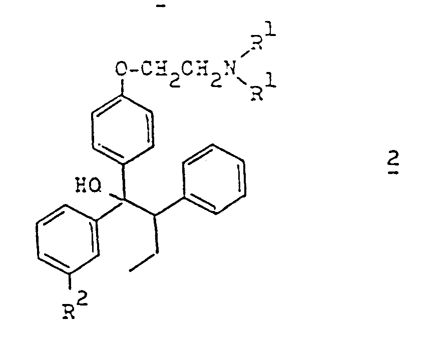 in der R¹ und R² die in der Formel 1 angegebene Bedeutung besitzen, mindestens 10 h bei einer Temperatur von 50° bis 60°C in mindestens 25 Gew.-% salzsaurem bzw. mindestens 40 Vol.-% schwefelsaurem Milieu unter Ausschluss von organischen Lösungsmitteln erhitzt und das Reaktionsprodukt nach üblichen Methoden aufarbeitet.

2. Verfahren nach Anspruch 1, dadurch **gekennzeichnet,** dass man 12 bis 16 h bei 50° bis 60°C erhitzt.

3. Verfahren nach Anspruch 1 oder 2, dadurch **gekennzeichnet,** dass die Salzsäurekonzentration 32 bis 37 Gew.-% beträgt.

4. Verfahren nach Anspruch 1 oder 2, dadurch **gekennzeichnet,** dass die Schwefelsäurekonzentration 45 bis 50 Vol.-% beträgt.

## Claims

1. Process for the preparation of trans-1,1,2-triphenyl-but-1-ene derivatives of the general formula 1 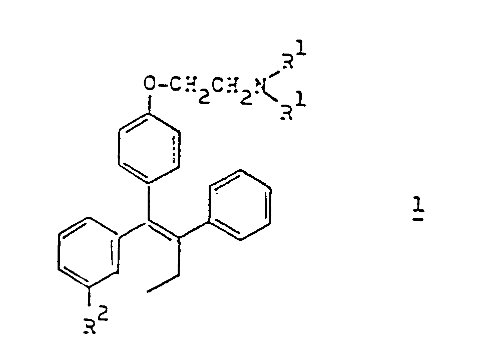 in which R¹ = CH₃, CH₂CH₃ and R² = H, OH,
characterised in that carbinols of the general formula 2 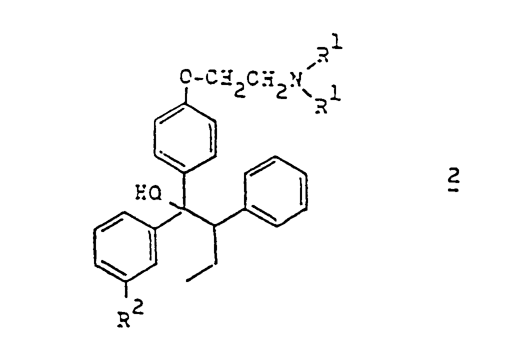 in which R¹ and R² have the meaning given in formula 1 are heated for at least 10 hours at a temperature of 50° to 60°C in at least 25 wt.% hydrochloric acid medium or at least 40 vol.% sulphuric acid medium with the exclusion of organic solvents and the reaction product is worked up by conventional methods.

2. Process according to claim 1, characterised in that heating is carried out for 12 to 16 hours at 50° to 60°C.

3. Process according to claim 1 or 2, characterised in that the hydrochloric acid concentration is 32 to 37 wt.%.

4. Process according to claim 1 or 2, characterised in that the sulphuric acid concentration is 45 to 50 vol.%.

## Revendications

1. Procédé de préparation de dérivés du trans-1,1,2-triphényl-but-1-ène répondant à la formule générale 1 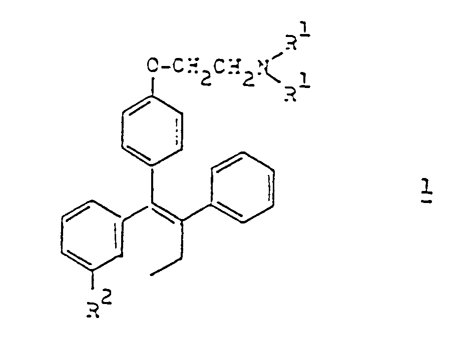 dans laquelle R¹ = CH₃, CH₂CH₃ et R² = H, OH,
caractérisé en ce qu'on chauffe des carbinols répondant à la formule générale 2 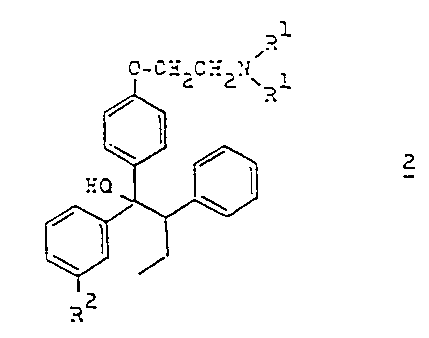 dans laquelle R¹ et R² ont la signification indiquée dans la formule 1, pendant au moins 10 heures, à une température de 50° à 60°C, en milieu chlorhydrique à 25 % en poids au moins ou en milieu sulfurique à 40 % en volume au moins, en l'absence de solvants organiques, et en ce qu'on poursuit le traitement du produit de la réaction par les procédés habituels.

2. Procédé selon la revendication 1, caractérisé en ce qu'on chauffe pendant 12 à 16 heures à 50 à 60°C.

3. Procédé selon les revendications 1 ou 2, caractérisé en ce que la concentration en acide chlorhydrique est de 32 à 37 % en poids.

4. Procédé selon les revendications 1 ou 2, caractérisé en ce que la concentration en acide sulfurique est de 45 à 50 % en volume.
